# EUROPÄISCHE PATENTANMELDUNG

(11) **EP 4 414 458 A1**
(43) Veröffentlichungstag der Anmeldung: **14.08.2024**
(21) Anmeldenummer: 23155303.3
(22) Anmeldetag: 07.02.2023
(51) Int. Cl.: C12Q 1/6806, G01N 33/68, C12N 15/10

(54) **COLLECT & EXTRACT - LÖSUNG FÜR DIE SAMMLUNG UND LAGERUNG BIOLOGISCHER PROBENTYPEN ZUR EXTRAKTION VON BIOMOLEKÜLEN**

(71) Anmelder: Senckenberg Gesellschaft Für Naturforschung, 60325 Frankfurt Am Main (DE)
(72) Erfinder: COCCHIARARO, Berardino, 63526 Erlensee (DE)
(74) Vertreter: Vossius & Partner Patentanwälte Rechtsanwälte mbB

(57) **Zusammenfassung**

Die vorliegende Erfindung betrifft eine Lösung zur Stabilisierung von Biomolekülen, die ein Guanidinsalz, bevorzugt 100 mM und 800 mM Guanidinsalz, am meisten bevorzugt 250 mM bis 400 mM; Natriumlaurylsulfat oder N-Laurylsarcosin, bevorzugt 0,1 - 2,5 Gew.-% Natriumlaurylsulfat oder N-Laurylsarcosin, am meisten bevorzugt 0,8 bis 1,2 Gew.-% Natriumlaurylsulfat oder N-Laurylsarcosin; und gegebenenfalls Ethanol, bevorzugt 3 bis 50 Vol.%, am meisten bevorzugt 4,5 bis 35,0 Vol.% umfasst.

## Beschreibung

Die vorliegende Erfindung betrifft eine Lösung zur Stabilisierung von Biomolekülen, die ein Guanidinsalz, bevorzugt 100 mM und 800 mM Guanidinsalz, am meisten bevorzugt 250 mM bis 400 mM; Natriumlaurylsulfat oder N-Laurylsarcosin, bevorzugt 0,1 bis 2,5 Gew.-% Natriumlaurylsulfat oder N-Laurylsarcosin, am meisten bevorzugt 0,8 bis 1,2 Gew.-% Natriumlaurylsulfat oder N-Laurylsarcosin; und gegebenenfalls Ethanol, bevorzugt 3 bis 50 Vol.%, am meisten bevorzugt 4,5 bis 35,0 Vol.% umfasst.

In der Beschreibung ist eine Vielzahl von Dokumenten, einschließlich Patentanmeldungen, Patenten und Bedienungsanleitungen verschiedener Hersteller zitiert. Der Offenbarungsgehalt jedes Einzelnen dieser Dokumente, obwohl für die Patentfähigkeit der vorliegenden Erfindung als nicht relevant angesehen, wird hiermit in den Offenbarungsgehalt der Anmeldung einbezogen. Insbesondere sind auch alle in diesen Dokumenten Bezug genommenen Dokumente in den Offenbarungsgehalt der Anmeldung einbezogen, und zwar in demselben Umfang, als wäre spezifisch jedes einzelne dieser Dokumente durch Bezugnahme in den Offenbarungsgehalt der vorliegenden Anmeldung einbezogen worden.

Genetische Analysen von Biomolekülen werden in vielen Fachbereichen der Medizin und Biologie wie auch im kommerziellen Kontext (z.B. Abstammungs- und Krankheits-Tests; siehe Diamandis et al., 2010 und de Groot, et al., 2021) zur Beantwortung diverser Fragestellungen durchgeführt. Sowohl in der medizinischen Diagnostik, der Forensik und Grundlagenforschung als auch in den Umweltwissenschaften sind sie unverzichtbar geworden. Die zu untersuchenden Probentypen variieren je nach Anwendungsgebiet und Fragestellung sehr stark, u.a. in ihrem Volumen, ihrer Beschaffenheit und beispielsweise ihrem Biomolekül-Gehalt, weshalb die verwendeten Produkte und Protokolle für deren Sammlung, Konservierung und Laboranalysen ebenso vielfältig sind. Für die medizinische DNA-Diagnostik werden häufig Blut, Speichel, (Tumor-) Gewebe, Stuhl und diverse Körperflüssigkeiten analysiert. Des Weiteren entwickelt sich in der Grundlagenforschung seit knapp 10 Jahren ein neues Forschungsfeld, welches Umwelt-DNA (environmental DNA; eDNA) untersucht (Thomsen und Willerslev, 2015), um einzelne bedrohte Arten oder gar ganze Artengemeinschaften zu erfassen. Die hauptsächlich genutzten Probenressourcen sind Wasser, Luft, Bodenproben und Sedimente. Zur Umwelt-DNA werden u.a. auch Hinterlassenschaften von Tieren, wie Urin, Kot und Haare gezählt. Im Gegensatz zur molekularen Analytik in der medizinischen Diagnostik, welche vorwiegend mittels frischgenommener Patientenproben durchgeführt werden kann, unterliegen Analysen von in der Umwelt gewonnenen Proben einer gewissen Limitierung, da die darin enthaltenen Biomoleküle mit der Zeit z.B. durch Witterung und Verwertung von Mikroorganismen zersetzt werden und dadurch in der Regel nur noch eine geringere Qualität und Quantität von Biomolekülen besitzen (Taberlet et al., 1999).

Die Konservierung von Proben, also die Erhaltung des Zustands ab dem Zeitpunkt ihrer Entnahme, ist insbesondere für Umweltproben, aber auch generell ein entscheidender Schritt in der Bioanalytik (Camacho-Sanchez et al, 2013). Häufig genutzte Konservierungsmethoden sind die Trocknung, das Gefrieren oder die Einlagerung in konservierenden Flüssigkeiten, wie hochprozentigem Ethanol, RNAlater (Qiagen GmbH, Hilden, Deutschland oder Thermo Fisher Scientific, Waltham, MA, USA) oder DNA/RNA-Shield (Zymo Research Europe GmbH, Freiburg, Deutschland).

Jedoch ist keine dieser konservierenden Flüssigkeiten und Lösungen zum Sammeln und Konservieren eines breiten Spektrums von Probentypen heterogener Konsistenz und Größe sowie geringem Biomolekülgehalt geeignet, insbesondere von Probentypen die DNA, RNA und Proteine enthalten, die alle konserviert und nach der Lagerung weiter analysiert werden sollen. Des Weiteren wäre es wünschenswert Biomoleküle wie DNA, RNA und Proteine über einen langen Zeitraum zu konservieren, selbst bei höheren Temperaturen als vergleichbare Lösungen dies erlauben.

Daher besteht weiter ein Bedarf an zusätzlichen konservierenden Flüssigkeiten und Lösungen und insbesondere solchen, die die verschiedensten Biomoleküle wie DNA, RNA und Proteine aus Umweltproben über einen langen Zeitraum und selbst bei höheren Temperaturen konservieren. Dieser Bedarf wird durch die vorliegen Erfindung befriedigt.

In einem ersten Aspekt betrifft die vorliegende Erfindung eine Lösung zur Stabilisierung von Biomolekülen, die ein Guanidinsalz, bevorzugt 100 mM und 800 mM Guanidinsalz, am meisten bevorzugt 250 bis 400 mM; Natriumlaurylsulfat oder N-Laurylsarcosin, bevorzugt 0,1 bis 2,5 Gew.-% Natriumlaurylsulfat oder N-Laurylsarcosin, am meisten bevorzugt 0,8 bis 1,2 Gew.-% Natriumlaurylsulfat oder N-Laurylsarcosin; und gegebenenfalls Ethanol, bevorzugt 3 bis 50 Vol.%, am meisten bevorzugt 4,5 bis 35,0 Vol.% umfasst.

Der Begriff "Lösung" bezeichnet ein homogenes Gemisch, das aus zwei oder mehr chemisch reinen Stoffen besteht. Sie enthält einen oder mehrere gelöste Stoffe (die Solute) und ein Lösungsmittel (das selbst eine Lösung sein kann), meist der Stoff, der in größerer Menge vorhanden ist. Die erfindungsgemäße Lösung beinhaltet zumindest ein Guanidinsalz, Natriumlaurylsulfat und Ethanol. Die erfindungsgemäße Lösung wird hierin auch als Collect & Extract-Lösung oder nur Collect & Extract bezeichnet.

Der Zweck "Stabilisierung von Biomolekülen" bedeutet, dass die Biomoleküle in der erfindungsgemäßen Lösung stabilisiert (oder auch konserviert) werden. Der Begriff "Stabilisierung" (oder auch "Konservierung") bezieht sich auf die strukturelle bzw. funktionelle Integrität von Biomolekülen und den hierauf basierenden biologischen Eigenschaften. Die für einen bestimmten Anwendungszweck erforderliche Aktivität eines Biomoleküls erfordert z.B. die weitgehende Beibehaltung seiner Struktur, insbesondere Primär-, Sekundär- und/oder (sofern vorliegend) Tertiärstruktur. Die Struktur wird nach Lagerung der Biomoleküle in der erfindungsgemäßen Lösung insbesondere so beibehalten, dass die Biomoleküle in weiteren biologischen oder biochemischen Assays im Wesentlichen genauso gut detektiert und/oder analysiert werden können wie vor der Lagerung der Biomoleküle in der erfindungsgemäßen Lösung. Die erfindungsgemäße Lösung macht die Biomoleküle somit lagerstabil. Dies wird untenstehend im Zusammenhang mit dem Verfahren des zweiten Aspekts der Erfindung weiter deutlich werden.

Der Begriff "Biomolekül" umfasst alle Formen von Biomolekülen, insbesondere Lipide, Kohlehydrate, Proteine, Polysaccharide und Nukleinsäuren. Ein Biomolekül ist eine in Lebewesen vorkommende chemische Verbindung. Biomoleküle in Lebewesen sind hauptsächlich aus Kohlenstoff, Wasserstoff, Sauerstoff, Stickstoff, Schwefel und Phosphor zusammengesetzt.

Guanidin (Cas-Nummer 113-00-8; CH₅N₃) ist eine chemische Verbindung an der Grenze zwischen anorganischer und organischer Chemie. Guanidin kann als Stickstoffanalogon der Kohlensäure aufgefasst werden. Die Substanz ist eine der stärksten organischen Basen und reagiert an der Luft spontan mit Luftfeuchtigkeit und Kohlenstoffdioxid zu Guanidiniumcarbonat, einem Guanidinsalz. Guanidin bildet mit Säuren Guanidininsalze, z. B. Guanidiniumchlorid, Guanidiniumthiocyanat, Guanidiniumnitrat und Guanidiniumcarbonat.

Die Konzentration des Guanidininsalzes in der Lösung ist mit steigender Präferenz 100 mM und 800 mM Guanidinsalz, 125 mM bis 600 mM, 200 mM bis 500 mM und 250 mM bis 400 mM.

Natriumlaurylsulfat (oder Natriumdodecylsulfat, auch SLS oder SDS; Cas -Nummer 151-21-3; C₁₂H₂₅NaO₄S) ist ein anionisches Tensid, also eine waschaktive Substanz, das als Detergens Verwendung findet, z. B. in Reinigungsmitteln und Zahnpasta. Im Stand der Technik der Biochemie und Biotechnologie findet es Anwendung als Denaturierungsmittel für Proteine und bei der Zelllyse. Die Wirkung auf Proteine basiert darauf, dass nichtkovalente Bindungen der Proteine unterbrochen und so deren Quartär- und Tertiärstruktur zerstört werden.

N-Laurylsarcosin (Cas -Nummer 97-78-9; C₁₅H₂₉NO₃) ist ein Derivat des Sarcosins. Seine Salze, vor allem das Natriumsalz, sind anionische Tenside, die als Detergentien Verwendung finden. Natrium-N-lauroylsarcosinat, Sarkosyl NL) besitzt im Gegensatz zu Natriumlaurylsulfat eine gute Löslichkeit in chaotropen Hochsalz-Lösungen und ist daher oftmals das Detergens der Wahl in guanidiniumhaltigen Zelllyse-Puffern aus dem Stand der Technik.

Die Menge von Natriumlaurylsulfat oder N-Lauroylsarcosin in der Lösung ist mit steigender Präferenz 0,1 bis 2,5 Gew.-%. 0,4 bis 2,0 Gew.-%, 0,6 bis 1,6 Gew.-% und 0,8 bis 1,2 Gew.-%. Natriumlaurylsulfat ist bevorzugt, da es in den Ausführungsbeispielen verwendet wird.

Die erfindungsgemäße Lösung enthält gegebenenfalls Ethanol. Ethanol (oder Ethylalkohol, EtOH, Äthanol, Äthylalkohol, umgangssprachlich (gewöhnlicher) Alkohol genannt; Cas-Nummer 64-17-5; C₂H₆O) ist ein aliphatischer, einwertiger Alkohol.

Die erfindungsgemäße Lösung enthält bevorzugt Ethanol. Für die vollständige Auflösung der Biomoleküle in einer Probe, wie etwa in der erfindungsgemäßen Lösung, ist Ethanol notwendig. Die angehängten Ausführungsbeispiele zeigen, dass sich Haare ohne Ethanol in der Lösung nicht vollständig auflösen. Auch wenn sich vermutlich noch genug Biomoleküle lösen, die gelagert und später analysiert werden können, ist ein vollständiges auflösen bevorzugt. Zudem ist Salzgehalt in der erfindungsgemäßen Lösung ohne Ethanol hoch, so dass die Lösung nach wenigen Minuten bei Raumtemperatur stark präzipitiert. Dies ist wiederum z.B. während DNA-Extraktionsverfahren hinderlich, da man die Probe nicht mehr pipettieren kann. Somit ist ohne Ethanol etwa keine direkte Überführung auf eine Silika-Säule möglich. Die Präzipitate lassen sich dann nur etwa durch Aufheizen auf über 30°C und eben die Zugabe von Ethanol lösen.

Die Menge von Ethanol in der Lösung ist mit steigender Präferenz 3 bis 50 Vol.%, 4 bis 40 Vol.% und 4,5 bis 35,0 Vol.%.

Die experimentellen Ergebnisse in den angehängten Ausführungsbeispielen zeigen, dass die Collect & Extract-Lösung nicht nur das Sammeln und Konservieren eines breiten Spektrums von Probentypen heterogener Konsistenz und Größe sowie geringem Biomolekülgehalt ermöglicht, sondern im Vergleich zu anderen Produkten aus dem Stand der Technik, wie DNA/RNA-Shield, nicht nur Nukleinsäuren, sondern auch Proteine konserviert, die dann in der Folge analysiert werden können. Des Weiteren konserviert Collect & Extract Proben länger und bei höheren Temperaturen als vergleichbare Lösungen. Durch ihre Zelllyse-Fähigkeit und Eigenschaft, die Biomoleküle unabhängig von der Umgebungstemperatur zu konservieren, vereinfacht ihre Verwendung die Transportlogistik der Proben vom Sammelort zum Analyselabor sowie die Bearbeitung von Proben für die Extraktion der Biomoleküle, wie der DNA, RNA oder Proteinen.

Sie ermöglicht aufgrund des flüssigen Zustands jeder Probe eine Einsparung der Manipulationsschritte des Laborpersonals, wodurch Kontaminationsrisiken und Arbeitszeit reduziert werden, als auch eine nahezu direkte Überführung von Proben in automatisierte Extraktionsprozesse. Übertragungsschritte können noch weiter reduziert werden, wenn Proben in Laborgerät-kompatiblen Gefäßen gesammelt werden. Eine wesentliche Anpassung von Labor-Protokollen bei der Verwendung von Collect & Extract ist nicht notwendig oder äußerst gering, da sie mit Silikasäulen- sowie Magnetic-Beads-basierten Extraktions-Kits und -Geräten der meisten Anbieter kompatibel ist.

Durch ihre Skalierbarkeit kann das eingesetzte Volumen von Proben für eine Extraktion von Biomolekülen anwendungsspezifisch angepasst werden, um die Erfolgswahrscheinlichkeit von Analysen oder Detektionen von geringem Spurenmaterial zu erhöhen.

Die Ergebnisse von äußerst schwierig analysierbaren Fischotterlosungen sowie im Freiland gesammelten Urinproben von Wölfen zeigen, dass durch die Verwendung von Collect & Extract, i) die Erfolgsquoten von molekularen Analysen solcher äußerst sensitiven und komplexen Umweltproben erhöht werden können, ii) die Konservierung über längere Zeiträume als bei vergleichbaren Lösungen, wie DNA/RNA-Shield, gewährleistet wird und iii) über Analysen von DNA und RNA hinaus auch Proteinanalysen ermöglicht. Die genetischen Analysen eines breiten Probenspektrums mittels Collect & Extract sowie einer exemplarischen Proteinanalyse werden in den angehängten Ausführungsbeispielen gezeigt.

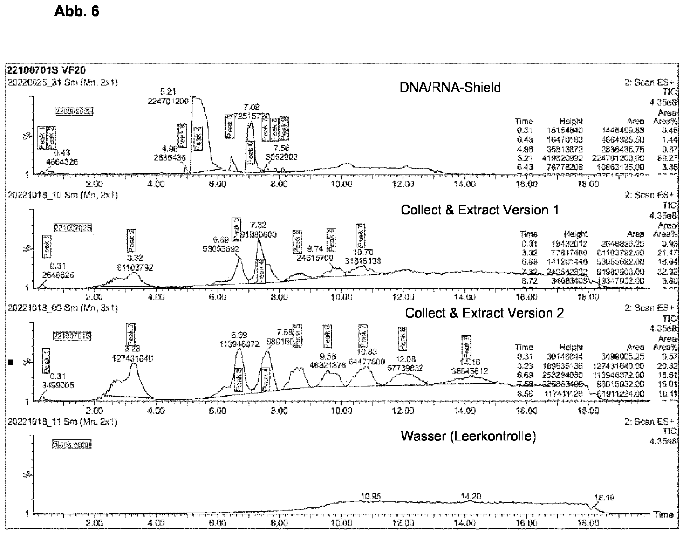

Bezüglich DNA/RNA-Shield wird auf die Herstellerangaben unter https://files.zymoresearch.com/protocols/_r1100-50_r1100-250_r1200-25_r1100-125_dna_rna_shield.pdf verwiesen. Gemäß den Herstellerangaben ist DNA/RNA-Shield nur zur Stabilisierung vom RNA und DNA nicht jedoch Protein geeignet. RNA und DNA sind in DNA/RNA-Shield zudem bei 5°C bis 30°C zwar mindestens für 30 Tage stabil und bei 35°C bis 40°C jedoch nur maximal 7 Tage. Die Daten in den angehängten Ausführungsbeispielen belegen hingegen, dass Collect & Extract auch Proteine stabil hält. DNA, RNA und Proteine sind über Monate hinweg (mind. die getesteten 7 Monate) stabil und Lagertemperaturen bis zu 52°C für 7 Wochen - sogar mit Proben, die vor der Lagerung bei 52°C bereits 7 Monate bei Raumtemperatur lagerten - wurden erfolgreich getestet. Ferner belegt das Beispiel 1.3 und die Abb. 6, dass die Inhaltsstoffe von DNA/RNA-Shield und Collect & Extract signifikant voneinander unterschiedlich sind. Somit leistet Collect & Extract einen neuen und erfinderischen Beitrag zum Stand der Technik.

In den Ausführungsbeispielen 1.1 und 1.2 sind die Herstellung und Verwendung der Collect & Extract Versionen 1 und 2 beschrieben. Die Version 2 entspricht einem Gemisch aus den kommerziell erhältlichen Puffern ATL und AL (beide Qiagen GmbH, Deutschland) und Ethanol 96% vol Ph. Eur. im Verhältnis 1:1:1. Die Version 1 wurde von den Erfindern selbst hergestellt, wie im Ausführungsbeispiel 1.1 beschrieben. Die wesentlichen Inhaltsstoffe von Collect & Extract Versionen 1 und 2 zur Stabilisierung von Biomolekülen sind ein Guanidinsalz, Natriumlaurylsulfat oder N-Laurylsarcosin sowie gegebenenfalls Ethanol. Das Beispiel 1.3 und die Abb. 6 belegen, dass die Inhaltsstoffe von Collect & Extract Versionen 1 und 2 nahezu identisch sind, mit der Ausnahme, dass Collect & Extract Version 2 zwei zusätzliche Inhaltsstoffe aufweist, die zur Stabilisierung von Biomolekülen nicht notwendig sind. Einer dieser Inhaltsstoffe ist Maleinsäure und der andere lässt sich anhand der Ergebnisse nicht festmachen. Die Collect & Extract Version 1 ist somit gegenüber der Version 2 bevorzugt, da sie mit weniger Inhaltsstoffen einfacher und kostengünstiger hergestellt werden kann.

Da Maleinsäure zur Stabilisierung von Biomolekülen nicht notwendig ist, umfasst oder beinhaltet die Lösung - entsprechend einer bevorzugten Ausführungsform des ersten Aspekts der Erfindung - keine Maleinsäure und/oder ein Addukt der Maleinsäure.

Entsprechend einer bevorzugten Ausführungsform des ersten Aspekts der Erfindung ist die Lösung eine wässrige Lösung.

Der Begriff "wässrige Lösung" bezeichnet eine Lösung, in der das Lösungsmittel für die Inhaltsstoffe der Lösung Wasser ist. In der erfindungsgemäßen Lösung sind zumindest ein Guanidinsalz, Natriumlaurylsulfat und Ethanol in der wässrigen Lösung gelöst.

Das Wasser in der wässrigen Lösung ist bevorzugt destilliertes Wasser. Destilliertes Wasser (auch lateinisch *Aqua destillata*) ist Wasser (H₂O), das durch Destillation von den im normalen Quellwasser oder Leitungswasser vorkommenden Ionen, Spurenelementen und anderen Verunreinigungen befreit wurde. Das destillierte Wasser ist bevorzugt mehrfach destilliertes Wasser, insbesondere zweifach destilliertes (bidestilliertes) Wasser (*aqua bidestillata*)*.*

Eine bevorzugte Ausführungsform des ersten Aspekts der Erfindung bestimmt, dass das Guanidinsalz Guanidinhydrochlorid oder Guanidinthiocyanat ist.

Guanidinhydrochlorid oder Guanidinthiocyanat sind beide als Reagenzien zur DNA-/RNA-Analyse kommerziell erhältlich. Beide Salze sind stark chaotrope Reagenzien, die effizient die dreidimensionale Struktur von Proteinen zerstören und sie somit denaturieren können. Guanidinhydrochlorid ist bevorzugt, da es in den angehängten Ausführungsbeispielen verwendet wird.

Gemäß einer bevorzugten Ausführungsform des ersten Aspekts der Erfindung umfasst die Lösung weiterhin einen Puffer, bevorzugt Tris-(hydroxymethyl)-aminomethanhydrochlorid (TRIS HCl).

Ein Puffer ist ein Stoffgemisch dessen pH-Wert (Konzentration der Oxoniumionen) sich bei Zugabe einer Säure oder einer Base wesentlich weniger stark ändert, als dies in einem ungepufferten System der Fall wäre. Die Wirkung des Puffers beruht auf der Umsetzung der durch die Säure bzw. Base zugeführten Oxoniumionen (H₃O⁺) bzw. der Hydroxidionen (OH⁻) zu schwachen Säuren bzw. Basen, die selbst nur wenig zur Bildung von H₃O⁺ bzw. OH⁻-Ionen neigen.

Der pH-Pufferbereich des verwendeten Puffers ist bevorzugt zwischen 6,0 und 10,0 und am meisten bevorzugt zwischen 7,0 und 9,0. Nicht-limitierende aber bevorzugte Beispiele solcher Puffer sind HEPES: 4-(2-Hydroxyethyl)-1-piperazinethanesulfonsäure (pH 6,8 bis 8,2); HEPPS: 4-(2-Hydroxyethyl)-piperazin-1-propansulfonsäure (pH 7,3 bis 8,7), Kohlensäure-Bicarbonat-System (pH 6,2 bis 8,6) und Tris-(hydroxymethyl)-aminomethanhydrochlorid (pH 7,0 bis 9,0). Weitere Puffer in dem genannten pH-Pufferbereich sind dem Fachmann bekannt.

Tris-(hydroxymethyl)-aminomethanhydrochlorid (TRIS HCl; oder Trometamol Hydrochlorid, Tris(hydroxymethyl)methylamin Hydrochlorid, 2-Amino-2-(hydroxymethyl)propan-1,3-diolhydrochlorid, THAM HCl, 2-Amino-2-(hydroxymethyl)-1,3-propandiol HCl, Tris(hydroxymethyl)methylammoniumchlorid) ist als Reagenz zur Stabilisierung von Pufferrezepturen und Elektrophorese von biologischen Molekülen bekannt. Tris(hydroxymethyl)aminomethan (Tris) ist ein Standardpuffer in den biologischen Wissenschaften mit einem pH-Pufferbereich zwischen 7,0 und 9,0. Es ist für elektrophoretische Studien und Kapillarelektrochromatographie geeignet.

Gemäß einer weiteren bevorzugten Ausführungsform des ersten Aspekts der Erfindung umfasst die Lösung weiterhin einen Chelator, bevorzugt Ethylendinitrilotetraessigsäure (EDTA) umfasst.

Als Chelatoren bezeichnet man organische oder selten anorganische Verbindungen, die zwei oder mehr freie Elektronenpaare aufweisen und damit mehr als eine koordinative Bindung mit einem zentralen (Metall-)lon eingehen können. Wobei das Metallion bevorzugt ein zweiwertiges Kation ist (z-B. Cu²⁺ oder Mg²⁺).

Nicht-limitierende aber bevorzugte Beispiele von Chelatoren sind Acetylaceton, Ethylendiamin, 2-(2-Aminoethylamino)ethanol, Diethylentriamin, Iminodiacetat, Triethylentetramin, Triaminotriethylamin, Nitrilotriacetat, Bis(salicyliden)ethylendiamin, Ethylendiaminotriacetat, Ethylendiamintetraacetat, Diethylentriaminpentaacetat, Triethylentetraminhexaacetat, 1,4,7,10-Tetraazacyclododecan-1,4,7,10-tetraacetat, Oxalat, Tartrat, Citrat, Dimethylglyoxim, 8-Hydroxychinolin, 2,2'-Bipyridin, 1,10-Phenanthrolin, Dimercaptobernsteinsäure und 1,2-Bis(diphenylphosphino)ethan.

Ethylendinitrilotetraessigsäure (EDTA) (CAS-Nummer 60-00-4, C₁₀H₁₆N₂O₈) komplexiert zweiwertige Metallkationen. EDTA ist einer der Bestandteile der TAE- und TBE-Puffer, die unter anderem bei der Gelelektrophorese, etwa zur Trennung von DNA-Fragmenten, Verwendung finden. Enzymlösungen werden oft mit EDTA versetzt, um der durch Schwermetallionen verursachten Hemmung der Enzymaktivität vorzubeugen.

Gemäß einer noch weiteren bevorzugten Ausführungsform des ersten Aspekts der Erfindung umfasst die Lösung weiterhin ein oder mehrere Detergenzien zur Zelllyse, bevorzugt Tween 20 und/oder Triton X-100.

Dem Fachmann sind zahlreiche Detergenzien zur Zelllyse bekannt. Nicht-limitierende aber bevorzugte Beispiele solcher Detergenzien sind Tween 20, Tween 80, NP40, Triton X-100, CHAPS-Detergenz (3-((3-Cholamidopropyl) dimethylammonio)-1-propansulfonat und Octylthioglucoside (OTG).

Tween 20 und/oder Triton X-100 sind bevorzugt. Beide solubilisieren Membranproteine und lysieren Zellen.

Tween 20 (oder Polysorbat 20, Polyoxyethylen(20)-sorbitan-monolaurat; Cas-Nummer 9005-64-5; C₅₈H₁₁₄O₂₆) ist ein nichtionisches Tensid.

Triton X-100 (oder Octoxinol 9, O-[4-(1,1,3,3-Tetramethylbutyl)phenoxy]polyethoxyethanol; Cas-Nummer 9002-93-1; C₁₄H₂₂O(C₂H₄O)n mit n = 9-10). ein ist p-tert-Octylphenol-Derivat mit einer Polyethylenglycol-Seitenkette aus 9 bis 10 Ethylenoxid-Einheiten. Es ist ein nichtionisches Tensid aus der Gruppe der Octylphenolethoxylate.

Entsprechend einer bevorzugten Ausführungsform des ersten Aspekts der Erfindung umfasst die Lösung weiterhin eine Proteinase, bevorzugt Proteinase K.

Proteinasen sind Enzyme, die Proteine spalten. Dabei lösen sie Peptidbindungen zwischen einzelnen Aminosäuren durch Hydrolyse. Sie gehören nach der EC-Klassifikation zu den Hydrolasen (Gruppe III).

Proteinase K (EC Nummer 3.4.21.64, Serinprotease) ist eine Proteinase aus den Schlauchpilzen *Engyodontium album* und *Acremonium strictum,* die zur Familie der Subtilisinähnlichen Serinproteasen gehört. Das Enzym greift Peptidbindungen sowohl an den Enden (Exopeptidase), als auch im Inneren (Endopeptidase) der Proteine an. Proteinase K wird für den Abbau von Proteinen in Zelllysaten und zur Freisetzung von Nukleinsäuren verwendet.

Entsprechend einer mehr bevorzugten Ausführungsform des ersten Aspekts der Erfindung enthält die Lösung Proteinase K in einer Menge von 2 bis 100 mAU/ml Lösung, bevorzugt 4 bis 80 mAU/ml Lösung und am meisten bevorzugt etwa 60 mAU/ml.

Proteinase K in diesem Konzentrationsbereich wird in den Ausführungsbeispielen für eine effiziente Freisetzung von Nukleinsäuren eingesetzt. 1 mg an Proteinase K ist äquivalent zu 30 mAU (AU = Anson-Unit oder Anson-Einheit). Eine AU ist definiert als die Enzymmenge, die 1,0 µmol (181 µg) Tyrosin pro Minute bei pH 7,5 und 37 °C aus Casein freisetzt.

Entsprechend einer anderen bevorzugten Ausführungsform des ersten Aspekts der Erfindung umfasst die Lösung weiterhin ein Reagenz zur Reduzierung der Disulfide, bevorzugt, Dithiothreitol (DTT) oder Mercaptoethanol.

Dem Fachmann sind zahlreiche Reagenzien zur Reduzierung der Disulfide bekannt. Nicht-limitierende aber bevorzugte Beispiele solcher Reagenzien sind Dithiothreitol (inkl. DL-Dithiothreitol, L-Dithiothreitol und D-Dithiothreitol), Dithioerythritol (DTE) und Mercaptoethanol.

Dithiothreitol (DTT) (oder Clelands Reagenz; Cas-Nummer 3483-12-3; C₄H1₀O₂S₂) findet Verwendung in der Proteinbiochemie. Es konserviert Proteine des Zellinneren in ihrer funktionalen Form, indem es die Oxidation von Sulfhydryl-Gruppen (SH-) zu Disulfidbrücken durch Luftsauerstoff verhindert.

Mercaptoethanol (oder 2-Sulfanylethanol, CAS-Nummer: 60 24 2, C₂H6OS) ist bevorzugt ß-Mercaptoethanol. Mercaptoethanol wird eingesetzt, um Proteine zu reduzieren und somit Disulfidbrücken zu zerstören.

Entsprechend einer mehr bevorzugten Ausführungsform des ersten Aspekts der Erfindung enthält die Lösung 1M DTT oder 14,3 M Mercaptoethanol in einer Menge von bis zu 20 Vol.%.

DTT oder Mercaptoethanol in diesem Konzentrationsbereich werden in den Ausführungsbeispielen für eine effiziente Verhinderung von Disulfidbrücken eingesetzt. 1M DTT in Wasser ist kommerziell erhältlich. Ebenso ist 14,3 M Mercaptoethanol (99% Reinheit) kommerziell erhältlich

In einem zweiten Aspekt betrifft die vorliegende Erfindung ein Verfahren zur Stabilisierung von Biomolekülen, umfassend (a) die Zugabe der Biomoleküle in eine Lösung nach dem ersten Aspekt der Erfindung, und (b) gegebenenfalls die Lagerung der Lösung mit den Biomolekülen bei -273°C bis 70°C, bevorzugt bei -20°C bis 30°C, bevorzugt für mindestens 14 Tage, mehr bevorzugt für mindestens 1 Monat und am meisten bevorzugt für mindestens 6 Monate.

Wie bereits im Zusammenhang mit dem ersten Aspekt der Erfindung erklärt, macht die Zugabe von Biomolekülen zur erfindungsgemäßen Lösung die Biomoleküle lagerstabil, so dass diese auch nach Lagerung ohne nennenswerte Einschränkungen nachgewiesen und/oder analysiert werden können.

Die angehängten Ausführungsbeispiele zeigen, dass Biomoleküle über viele Monate hinweg in der erfindungsgemäßen Lösung bei Raumtemperatur stabil sind, da anschließende Nachweise und/oder Analysen der Biomoleküle ohne nennenswerte Einschränkungen funktionieren. Die angehängten Ausführungsbeispiele zeigen ebenso, dass die erfindungsgemäße Lösung die Stabilität der Biomoleküle sogar bei Temperaturen gewährleistet, die deutlich über Raumtemperatur (über 50°C) liegen.

Die Lagertemperatur ist mit steigender Präferenz -273°C bis 70°C, -20°C bis 100°C, -20°C bis 55°C, -20°C bis 30°C, und 0°C bis 30°C.

Die Lagerdauer ist mit steigender Präferenz mindestens 1 Tag, mindestens 3 Tage, mindestens 7 Tage, mindestens 14 Tage, mindestens 1 Monat, mindestens 3 Monate, mindestens 6 Monate, mindestens 1 Jahr, mindestens 1,5 Jahre, mindestens 2 Jahre und mindestens 5 Jahre.

Die Lagerung erfolgt bevorzugt unter Ausschluss von Licht.

Entsprechend einer bevorzugten Ausführungsform des zweiten Aspekts der Erfindung werden im Schritt (a) der Lösung Zellen zugegeben, die Biomoleküle umfassen.

Im Zusammenhang mit dieser bevorzugten Ausführungsform ist es besonders bevorzugt, dass die Lösung Detergenzien zur Zelllyse umfasst, insbesondere Tween 20 und/oder Triton X-100. Dies sorgt dafür, dass die Zellen während der Lagerung lysiert werden und die Biomoleküle somit in der Lösung freigesetzt werden. Die Freisetzung macht die Biomoleküle für weitere molekularbiologische Analysen zugänglich.

Entsprechend einer mehr bevorzugten Ausführungsform des zweiten Aspekts der Erfindung sind diese Zellen Teil einer biologischen Probe, wobei die biologische Probe bevorzugt Blut, Plasma, Serum, Urin, Speichel, Lymphe, Liquor, Sperma, Haut (-schuppen), Stuhl, Haare, Gewebe, Wasser, Pflanzenmaterial oder Bodenmaterial ist.

Die Stabilisierung von Biomolekülen in biologischen Proben ist von hoher praktischer Relevanz, bis diese molekularbiologischen Analysen zugeführt werden. Anwendungsfelder sind z.B. in der medizinischen und klinischen Diagnostik, in der Forensik, in der Pharmazie bei der Entwicklung und Evaluierung von Arzneimitteln, in der Lebensmittelanalytik sowie bei der Überwachung der Lebensmittelherstellung, in der Agrarwirtschaft bei der Züchtung von Nutzpflanzen und Nutztieren sowie in der Umweltanalytik zu finden. Hier liegt der Zeitpunkt der Entnahme der biologischen Probe oftmals notwendigerweise deutlich vor der Analyse, so dass eine stabile Lagerung bis zur weiteren Analyse notwendig ist.

Abhängig von der Natur der biologischen Probe kann die erfindungsgemäße Lösung angepasst werden, so dass neben der Stabilisierung und Zelllyse noch weitere Vorteile bestehen. So sind zum Beispiel DTT und Proteinase K für Haare und Gewebe von Vorteil, da diese während der Lagerung dann vollständig gelöst werden.

Entsprechend einer anderen bevorzugten Ausführungsform des zweiten Aspekts der Erfindung werden die Biomoleküle und die Lösung im Schritt (b) in einem fest verschlossen Gefäß, bevorzugt einem fest verschlossenen Röhrchen, gelagert.

Das fest verschlossene Gefäß, bevorzugt Röhrchen, schützt die Biomoleküle vor Umwelteinflüssen während der Lagerung wie etwa Schimmelbildung oder Kontamination mit externen Biomolekülen. Das Gefäß, bevorzugt Röhrchen, hat bevorzugt ein Volumen zwischen 1,5 und 50 ml.

Entsprechend einer weiteren bevorzugten Ausführungsform des zweiten Aspekts umfasst das Verfahren weiterhin (c) die Analyse der Biomoleküle nach der Lagerung im Schritt (b) umfasst, bevorzugt mit Silika-Säulen oder magnetischen Kügelchen.

Die Analyse der Biomoleküle wird in den angehängten Ausführungsbeispielen illustriert. Durch die Analyse des Transkriptoms, speziell der mRNA in Zellen, lassen sich die Aktivitäten von Genen direkt bestimmen. Die quantitative Analyse von Transkriptmustern (mRNA-Mustern) in Zellen durch moderne molekularbiologische Methoden, wie z. B. Echtzeit-Reverse-Transkriptase-PCR ("Real time RT PCR") oder Genexpressions-Chip-Analysen ermöglicht z. B. die Erkennung fehlerhaft exprimierter Gene, wodurch z. B. Stoffwechselkrankheiten, Infektionen oder eine etwaige Prädisposition für eine Krebserkrankung erkannt werden können.

Die Analyse des Genoms durch molekularbiologische Methoden, wie z.B. PCR, RFLP, AFLP oder Sequenzierung ermöglicht z. B. den Nachweis genetischer Defekte oder die Bestimmung des HLA-Typs sowie anderer genetischer Marker. Die Analyse genomischer DNA und RNA wird auch zum direkten Nachweis von infektiösen Erregern, wie Viren, Bakterien usw. eingesetzt.

Die Analyse des Proteoms durch molekularbiologische Methoden, wie z.B. Western Blot, Enzyme-linked Immunosorbent Assay (ELISA), Massenspektrometrie oder Sequenzierung ermöglicht ebenso z. B. den Nachweis genetischer Defekte oder Erkrankungen mit aberranten Proteinen.

Silika-Säulen, insbesondere Silika-basierte HPLC Säulen als auch magnetischen Kügelchen sind kommerziell erhältlich und wurden speziell zur Analyse von Biomolekülen entwickelt. So können z. B. Antikörper oder andere Liganden, die an einzelne Biomoleküle oder an Oberflächenstrukturen ganzer Zellen binden, an Silika-Säulen oder magnetische Kügelchen gekoppelt werden, ihre spezifischen Bindungspartner auch in komplexen Mischungen erkennen und anschließend mit Hilfe eines Elektromagneten herausgefischt werden.

Entsprechend einer bevorzugten Ausführungsform des ersten und zweiten Aspekts umfassen die Biomoleküle DNA, RNA und Proteine.

Aus dem Stand der Technik ist bekannt, dass es schwierig ist, in einer biologischen Probe gleichzeitig das Genom, das Transkriptom, und das Proteom - also DNA, RNA und Proteine - zu stabilisieren. Um z.B. eine Probe vor einem RNA Abbau durch die ubiquitären RNAsen zu schützen, wäre es sinnvoll sämtliche Enzyme im Medium (und damit auch alle RNAsen) zu denaturieren. Dies würde jedoch dem oben gesteckten Ziel entgegenlaufen, da hierdurch das Proteom massiv beeinträchtigt würde. Dasselbe würde gelten, wenn man versuchen würde, Proteasen zu denaturieren, um den proteasevermittelten Proteinabbau zu verhindern. Umgekehrt werden durch eine Maßnahme, die das Proteom einer Probe möglichst unbeeinträchtigt lässt, auch die ubiquitären RNAsen geschont, so dass eine Beeinträchtigung des Transkriptoms zu befürchten ist.

Die angehängten Ausführungsbeispiele zeigen, dass die erfindungsgemäße Lösung und somit gleichermaßen das erfindungsgemäße Verfahren DNA, RNA und Proteine stabilisieren, was technisch vorteilhaft ist. Nach der Lagerung können DNA, RNA und Proteine weiter analysiert werden.

In einem dritten Aspekt betrifft die vorliegende Erfindung ein Kit, umfassend die Lösung nach dem ersten Aspekt, ein oder mehrere Gefäße, bevorzugt ein oder mehrere Röhrchen, die fest verschlossen werden können, und gegebenenfalls Instruktionen zur Verwendung der Kits.

Auch hier schützt das Gefäß, bevorzugt Röhrchen, zum späteren Schutz von Biomolekülen vor Umwelteinflüssen während der Lagerung wie etwa Schimmelbildung oder Kontamination mit externen Biomolekülen. Das Gefäß, bevorzugt Röhrchen, hat bevorzugt ein Volumen zwischen 1,5 und 50 ml.

Das Gefäß, bevorzugt Röhrchen, kann separat von der Lösung nach dem ersten Aspekt im Kit vorliegen oder befindet sich bevorzugt bereits vorgelegt im Gefäß, bevorzugt Röhrchen. Die Instruktionen zur Verwendung der Kits geben bevorzugt Anweisung zur Verwendung des Kits zur Stabilisierung von Biomolekülen. Somit ist das Kit ebenso ein Kit zur Stabilisierung von Biomolekülen.

Sofern nicht anders definiert, haben alle hierin verwendeten technischen und wissenschaftlichen Begriffe die gleiche Bedeutung, wie sie von einem Fachmann auf dem Gebiet, zu dem diese Erfindung gehört, allgemein verstanden werden. Im Falle von Konflikten ist die Patentschrift einschließlich der Definitionen maßgebend.

Es versteht sich, dass die oben im Zusammenhang mit dem ersten Aspekt der vorliegenden Erfindung beschriebenen Definitionen und Ausführungsformen der Erfindung, so dies möglich ist, auch für den zweiten, dritten, vierten und jeden weiteren Aspekt der vorliegenden Erfindung entsprechend gelten.

Hinsichtlich der in dieser Beschreibung, insbesondere in den Ansprüchen, beschriebenen Ausführungsformen ist beabsichtigt, dass jede in einem abhängigen Anspruch erwähnte Ausführungsform mit jeder Ausführungsform jedes (unabhängigen oder abhängigen) Anspruchs, von dem dieser abhängige Anspruch abhängt, kombiniert werden kann. Im Falle eines unabhängigen Anspruchs 1, der 3 Alternativen A, B und C angibt, eines abhängigen Anspruchs 2, der 3 Alternativen D, E und F angibt, und eines Anspruchs 3, der von den Ansprüchen 1 und 2 abhängig ist und 3 Alternativen G, H und I angibt, ist es zum Beispiel so zu verstehen, dass die Beschreibung eindeutig Ausführungsformen offenbart, die den Kombinationen A, D, G entsprechen; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, sofern nicht ausdrücklich anders angegeben. Dies trifft auch für die Kombination verschiedener Alternativen zu, die in unterschiedlichen Unteransprüchen genannt sind.

In ähnlicher Weise und auch in den Fällen, in denen in unabhängigen und/oder abhängigen Ansprüchen keine Alternativen genannt werden, gilt jede Kombination von Gegenständen, die durch diese Ansprüche abgedeckt werden, als ausdrücklich offenbart, wenn sich abhängige Ansprüche auf eine Vielzahl vorhergehender Ansprüche beziehen. Im Falle eines unabhängigen Anspruchs 1, eines abhängigen Anspruchs 2, der auf Anspruch 1 zurückverweist, und eines abhängigen Anspruchs 3, der sowohl auf Anspruch 2 als auch auf Anspruch 1 zurückverweist, ist beispielsweise die Kombination der Gegenstände der Ansprüche 3 und 1 ebenso klar und eindeutig offenbart wie die Kombination der Gegenstände der Ansprüche 3, 2 und 1. Falls ein weiterer abhängiger Anspruch 4 vorhanden ist, der sich auf einen der Ansprüche 1 bis 3 bezieht, folgt daraus, dass die Kombination der Gegenstände der Ansprüche 4 und 1, der Ansprüche 4, 2 und 1, der Ansprüche 4, 3 und 1 sowie der Ansprüche 4, 3, 2 und 1 klar und eindeutig offenbart ist.

Die Erfindung wird hierin nur beispielhaft unter Bezugnahme auf die beigefügten Abbildungen und zum Zweck der Veranschaulichung der bevorzugten Ausführungsformen der vorliegenden Erfindung beschrieben.

Die Abbildungen zeigen:
**Abbildung 1****:** Überprüfung der DNA-Integrität von Speichelproben nach drei, sechs, 53 und 69 Tagen Lagerung in der Collect & Extract-Lösung (Version 1).
**Abbildung 2****:** Überprüfung der DNA-Integrität von Blutproben nach 14 Tagen, 21 Tagen und 28 Tagen Lagerung bei Raumtemperatur in der Collect & Extract-Lösung (Version 2).
**Abbildung 3****:** STR-Analyse von Wisent-Blutproben nach 21 Tagen Lagerung bei Raumtemperatur in der Collect & Extract-Lösung (Version 2). Alle fünf Marker liefern robuste, sehr gut auswertbare Rohdaten.
**Abbildung 4****:** PCR-Erfolg von vier Wasserproben für drei Organismengruppen
   Bei 1-4 handelt es sich um die vier Wasserproben. N1 und N2 bezeichnen die PCR-Negativ-Kontrollen.
**Abbildung 5****:** Sodiumdodecylsulphat-Polyacrylamid-Gelelectrophorese (SDS-PAGE) von Proteinen aus Wisentblutproben: 1, PageRuler-Proteinleiter; 2-4, drei Wisentblutproben gelagert in Collect & Extract-Lösung (Version 1) für 15 Tage bei 52°C; 5, eine Wisentblutprobe gelagert in Collect & Extract-Lösung (Version 1) für 15 Tage bei Raumtemperatur; 6, Extraktionskontrolle (blank); 7, kommerziell erhältliches BSA gelagert in Collect & Extract-Lösung (Version 1) für 15 Tage bei Raumtemperatur.
**Abbildung 6****:** Auftrennung der Lösungen DNA/RNA-Shield sowie Collect & Extract beider Versionen 1 und 2 mittels Flüssigchromatographie mit Massenspektrometriekopplung im positiven lonisierungsmodus (LC-MS-ESI, durchgeführt bei Fa. Laus GmbH, Kirrweiler, Deutschland) zur Ermittlung der Ingredienzenprofile.

Die Beispiele erläutern die Erfindung.

### Beispiel 1: Material und Methoden

### 1.1 Herstellung der Collect & Extract-Lösung: Version 1

Als Vorstufe der *Collect & Extract*-Lösung wurden zuerst zwei einzelne Komponenten hergestellt. Für Komponente 1 wurde eine 20%ige Natriumlaurylsulfat -Lösung (ROTI^{®}Stock 20 % SDS, BioScience Grade, ready-to-use, sterilfiltriert, Carl Roth GmbH + Co. KG, Deutschland) mit doppelt destilliertem Wasser (Carl Roth GmbH + Co. KG, Deutschland) 1:8 verdünnt, woraus eine 2,5 %ige SDS-Lösung resultierte. Komponente 2 bestand aus 30mM Tris-(hydroxymethyl)-aminomethanhydrochlorid (Carl Roth GmbH + Co. KG, Deutschland), 30mM Ethylendinitrilotetraessigsäure (Carl Roth GmbH + Co. KG, Deutschland), 5% Tween 20 (AppliChem GmbH, Deutschland), 0,5% Triton X-100 (AppliChem GmbH, Deutschland) und 800mM Guanidin-HCl (bei Herstellung der Komponente 2 wurde ein Zehntel des Gesamtvolumens einer 8M Guanidin-HCL-Lösung, Thermo Fisher Scientific, Waltham, MA, USA, verwendet), welche in doppelt destilliertem Wasser (Carl Roth GmbH + Co. KG, Deutschland) gelöst und mit Natronlauge auf pH 8 eingestellt wurde. Beide Komponenten und Ethanol 96% vol *Ph. Eur.* (VWR International GmbH, Deutschland) wurden im Verhältnis von 1:1:1 zusammengegeben.

### 1.2 Herstellung der Collect & Extract-Lösung Version 2 (mit Komponenten der Fa. Qiagen, Hilden, Deutschland):

Die kommerziell erhältlichen Puffer ATL und AL (beide Qiagen GmbH, Deutschland) und Ethanol 96% vol *Ph. Eur.* (VWR International GmbH, Deutschland) wurden im Verhältnis von 1:1:1 zusammengegeben.

### 1.3. Ingredienzenprofile der Lösungen DNA/RNA-Shield und Collect & Extract Versionen 1 und 2

Die Ingredienzenprofile der Lösungen DNA/RNA-Shield und *Collect & Extract* beider Versionen, die mittels Flüssigchromatographie mit Massenspektrometriekopplung im positiven lonisierungsmodus (LC-MS-ESI) aufgetrennt wurden, zeigen, dass DNA/RNA-Shield größtenteils aus anderen Inhaltsstoffen als *Collect & Extract* besteht (Abb. 6).

Drei Peaks (bei Retentionszeiten von 0,31, -6,6 und -7,3) wurden zwar auch in *Collect & Extract* detektiert, viele Peaks jedoch lediglich in der jeweils anderen Lösung (bei Retentionszeiten von 4.96 und 5,21 in DNA/RNA-Shield sowie von -3,3, -8,6, -9,6 und -10,75 in *Collect & Extract* beider Versionen). Auch die über die Peak Area abgeleiteten Mengenanteile dieser sich unterscheidenden Peaks sind verhältnismäßig hoch und deuten zusätzlich auf wesentliche Unterschiede in der Komposition von DNA/RNA-Shield und *Collect & Extract* hin.

Die *Collect & Extract* Versionen 1 und 2 sind bis auf zwei zusätzlich detektierte Peaks in Version 2 (bei Retentionszeiten von 12,06 und 14,16) nahezu identisch zusammengesetzt. Bei einem der beiden zusätzlich nachgewiesenen Peaks in *Collect & Extract* Version 2 handelt es sich um Maleinsäure bzw. um ein Addukt dieser, da Maleinsäure Bestandteil des Puffers AL und somit der *Collect& Extract* Version 2 ist und bei der Herstellung der *Collect & Extract* Version 1 nicht zugefügt wird. Der Bestandteil, der dem zweiten Peak zugrunde liegt, bleibt indes unbekannt. Allerdings zeigen die experimentellen Daten, dass sowohl die Maleinsäure als auch die unbekannte Komponente für die Stabilisierung von Biomolekülen von *Collect & Extract* nicht notwendig sind.

### 1.4 Reagenzien mit optionaler Zugabe zu Version 1 und 2:

Proteinase K, 20 mg/ml (Qiagen, Deutschland) wurde je nach Volumen der verwendeten *Collect & Extract*-Lösung bis zu einem 15%igem Anteils des Gesamtvolumens bei allen folgenden Experimenten hinzugegeben, maximal aber 120µl.

1M DL-Dithiothreitol (DTT)-Lösung, BioUltra, for molecular biology in H2O (Merck KGaA, Deutschland) wurde für die vollständige Auflösung von Muskelgewebe oder Haarproben bis zu einem 20%igem Anteil am Gesamtvolumen der verwendeten *Collect & Extract*-Lösung hinzugegeben.

### 1.5 Variation des Ethanolanteils

Der Ethanolanteil in der *Collect & Extract*-Lösung beider Versionen wurde für die Ermittlung einer effizienteren Lysefähigkeit von härteren Probentypen, wie Muskelgewebe- und Haarproben, variiert. In allen Varianten wurden Proteinase K und 1M DTT-Lösung hinzugegeben (siehe in 2.7).

### 1.6 DNA-Extraktion

Die DNA aller Umweltproben (Haare, Urin, Lösungen, Wasserproben) wurde in einem eigens für die Prä-PCR-Behandlung nichtinvasiv gesammelter und forensischer Umweltproben eingerichteten Reinstlabor unter Verwendung des QIAamp DNA Investigator Kit und des QIAamp Fast DNA Stool Mini Kit (beide Qiagen, Deutschland) nach Herstellerangaben vorsichtig extrahiert. Zur Testung auf Kompatibilität mit anderen Silika-Säulen-basierten DNA-Extraktionskits wurden das PureLink DNA-Minikit (Thermo Fisher Scientific, Waltham, MA, USA) sowie das NucleoSpin DNA Stool Mini kit (Macherey-Nagel, Düren, Deutschland) verwendet. Des Weiteren wurde die Testung auf Kompatibilität mit der ebenso weitläufig verwendeten Magnetic-Bead-Technologie mittels des EZ2 Connect-Extraktionsroboters und des EZ1&2 Virus Mini Kit v2.0 (beide Qiagen, Deutschland) durchgeführt. Die KompatibilitätsTestungen mit anderen Silika-Säulen-basierten DNA-Extraktionskits wurden in die beschleunigten Haltbarkeitstestungen integriert und mittels drei Fischotterkotproben durchgeführt (siehe Details unter 2.7. Kot). Zur Kontaminationsvermeidung wurden nur DNAfreie und sterile Gerätschaften verwendet sowie Handschuhe und weitere Verbrauchsmaterialien nach jeder Probe gewechselt. Durch das Mitführen von Extraktions- und PCR- (Polymerase-kettenreaktion)kontrollen wurde auf mögliche Kontaminationen im DNA-Extraktionsprozess und im Prä-PCR-Setup gemonitort. Die DNA invasiv gesammelter Probentypen, wie Speichel, Blut und Muskelgewebe, wurde in einem separaten Labor für Proben mit hohem DNA-Gehalt mittels des DNeasy Blood & Tissue Kit (Qiagen, Hilden, Deutschland) isoliert. Im Gegensatz zu den Standard-Protokollen aller verwendeten DNA-Extraktionskits wurde bei allen Experimenten das Lsyat nach 10-minütiger Inkubation bei 56°C direkt auf die Silika-Säule transferiert. Alle weiteren Schritte wurden nach Standardprotkoll durchgeführt. Bei Kot- und Urinproben wurde zur effektiveren Entfernung von potentiellen PCR-Inhibitoren jeder Waschschritt doppelt durchgeführt. Das Elutionsvolumen wurde Probentyp-spezifisch angepasst und wird in 1.7 angegeben.

### 1.7 Proteinextraktion und SDS-PAGE

Je 300 µl Blutserum von vier Wisenten (*Bos bonasus*) und 280 µl einer kommerziell erhältlichen BSA-Lösung (Bovine Serum Albumin; 20mg/ml; New England Biolabs GmbH, Deutschland) als Positiv-Kontrolle wurden in 1,2 ml Collect & Extract-Lösung (Version 1) gegeben. Drei der Proben wurden anschließend für 15 Tage bei 52°C und eine Probe sowie die Positivkontrolle mit kommerziell erhältlichem BSA bei Raumtemperatur gelagert. Vor der Extraktion der Proteine wurden die Proben für 30 min bei 56°C inkubiert und die darin befindlichen Proteine mittels AllPrep DNA/RNA/Protein Mini Kit (Qiagen, Deutschland) isoliert. Hierzu wurde das Protokoll "Simultaneous Purification of Genomic DNA, Total RNA and Total Protein from Animal and Human Cells" mit folgenden Anpassungen genutzt: Nach 30-minütiger Inkubation bei 56°C wurden als Input 350 µl des Lysats jeder Probe direkt zur DNAbindenden Säule (sonstige Lösungen, z.B. Nutzung des Lysepuffers RLT, und die vorbereitenden Schritte für die Lyse der Proben aus dem Standardprotokoll wurden nicht genutzt bzw. übersprungen) gegeben und der Durchfluss nach Zentrifugation ohne die nach Protokoll benötigte Zugabe von 250 µl Ethanol auf die RNA-bindende Säule überführt. Ab diesem Schritt erfolgte die Proteinextraktion gemäß Standardprotokoll. Für die darauffolgende Sodiumdodecylsulphat-Polyacrylamid-Gelelectrophorese (SDS-PAGE) wurde das Bolt^{™} Bis-Tris Plus Mini Gel Welcome Pack, 4-12% (Thermo Fisher Scientific, Waltham, MA, USA) verwendet. Zu den in 100 µl ALO-Puffer gelösten Proteinen wurden vor der Elektrophorese 35 µl des 4x LDS Probenpuffers (Bestandteil des Welcome Packs; siehe oben) hinzugefügt, je Probe 1 µl sowie 5 µl der vorgefärbten PageRuler-Proteinleiter (ebenfalls Bestandteil des Welcome Packs; Referenzbanden von 10 bis 190 kDa) auf das Bis-Tris-Gel aufgetragen und unter Verwendung eines PowerEase^{™} Touch 120-W-Netzteils (230 V AC; Thermo Fisher Scientific, Waltham, MA, USA) sowie integriertem Standardprogramm (NuPAGE^{™} Bis-Tris gels with MES) die Proteine elektrophoretisch aufgetrennt. Nach der Elektrophorese wurde das Gel über Nacht in 100 ml SimplyBlue^{™} SafeStain (Thermo Fisher Scientific, Waltham, MA, USA) gefärbt und anschließend in 100 ml destilliertem Wasser (Carl Roth, Deutschland) über eine weitere Nacht entfärbt. Die Größen der erhaltenen Proteinbanden wurden anhand der mitgelaufenen PageRuler-Proteinleiter abgeglichen.

### 1.8 Verwendete Probentypen und experimentelle Konfiguration

**Speichel (Mundschleimhautabstrich):** 11 Personen (*Homo sapiens*) führten mittels zweier Wattestäbchen (ROTILABO^{®} Holz steril, Carl Roth, Deutschland) jeweils eine Selbstbeprobung ihrer Mundschleimhaut durch. Danach wurden die entnommenen Proben direkt in 2,0 ml Safe-Lock-Tubes (Eppendorf, Deutschland) gelagert, welche mit 830 µl der Collect & Extract-Lösung (Version 1) und 120µl Proteinase K befüllt waren. In verschiedenen Zeitabständen nach Lagerung (3 Tage, 6 Tage, 53 Tage und 69 Tage) wurde die DNA von je zwei bis drei Proben (500 µl Lysat-Input; 100µl Eluat) mittles DNeasy^{®} Blood & Tissue Kit (Qiagen, Hilden, Deutschland) extrahiert und durch ein 1,4 %iges Agarosegel (150 ml ROTIPHORESE^{®}1x TAE, Carl Roth, Deutschland; 2,1 g Gel Pilot LE Agarose, Qiagen, Hilden, Deutschland, 15 µl GelRed^{®} Nucleic Acid Gel Stain, 10000x in Wasser, Biotium Inc., USA) unter Verwendung einer 1kb DNA-Leiter (Carl Roth, Karlsruhe, Deutschland) auf Stabilität der DNA-Integrität geprüft.

**Muskelgewebe und Haare:** Eine Gewebeprobe eines im Rahmen des genetischen Wolfmonitorings in Deutschland geborgenen (siehe DBBW - Dokumentations- und Beratungsstelle des Bundes zum Thema Wolf; https://www.dbb-wolf.de/) Wolf-Totfundes (*Canis lupus*) und eine über die Lockstockmethode (Hupe and Simon, 2007; Steyer et al., 2013) gesammelte Wildkatzenhaarprobe (*Felis silvestris*) wurden zur Testung der Lysefähigkeit der *Collect & Extract*-Lösung von härteren Probentypen in je 900 µl *Collect &* Extract-Lösung aufgeteilt, wovon der Volumenanteil 20% 1M DTT-Lösung und 10% µl Proteinase K betrug, und bei Raumtemperatur gelagert wurde. Die Gewebeprobe wurde in etwa gleichgroße Stücke à -20-30 mg geschnitten und in *Collect* & Extract-Lösung beider Versionen mit unterschiedlichem Ethanolanteil gelagert. Die Haarprobe wurde in *Collect & Extract-*Lösung der Version 2 mit unterschiedlichem Ethanolanteil aufgeteilt, so dass in jedem Anstaz 5-10 Haare mit Wurzel vorlagen. Für jede *Collect & Extract*-Lösungsversion wurde ein Replikat ohne Etahnol (1:1:0), mit einem Zehntel-Anteil (1:1:0,1) und halbem Anteil (1:1:0,5) Ethanol gegenüber der Komponenten 1 und 2 getestet (siehe 2.1 und 2.2). Die DNA der Gewebeprobe (600 µl Lysat-Input; 100µl Eluat) wurde nach 48 Tagen mittels DNeasy^{®} Blood & Tissue Kit (Qiagen, Deutschland) und die der Haarprobe nach sechs Tagen mittels QIAamp DNA Investigator Kit (Qiagen, Deutschland) (900 µl Lysat-Input; 80µl Eluat in zwei Schritten je 40 µl) extrahiert und anschließend einer artspezifischen STR-Analyse (Short-Tandem-Repeat; nach Jarausch et al. (2021) für die Tierart Wolf bzw. nach Steyer et al. (2013) für die Tierart Wildkatze unterzogen.

**Blut:** Von 11 Wisenten (*Bos bonasus*)*,* die im Rahmen eines Auswilderungsprojekts in Rumänien (für Informationen zum Projekt siehe https://www.wwf.de/themenprojekte/bedrohte-tier-und-pflanzenarten/wisente/umzug-der-wisente) invasiv beprobt wurden, wurden 100 µl Vollblut in 900µl der *Collect & Extract*-Lösung der Version 2 unter Zugabe von 100µl Proteinase K bei Raumtemperatur gelagert. Die DNA von je drei bis vier Proben (900 µl Lysat-Input; 100µl Eluat) wurde in drei Zeitabständen (14 Tage, 21 Tage und 28 Tage) mittels DNeasy^{®} Blood & Tissue Kit (Qiagen, Deutschland) extrahiert und mittels Agarosegel unter Verwendung einer 1kb DNA-Leiter auf Stabilität der DNA-Integrität geprüft. Des Weiteren wurden die DNA-Extrakte einer artspezifischen STR- sowie SNP (Single Nucleotide Polymorphism)-Analyse nach Wehrenberg et al., *in prep* unterzogen.

**Kot:** 12 komplette Kotproben von Fischottern (*Lutra lutra*) wurden im Dezember 2021 in 15 ml der *Collect & Extract*-Lösung der Version 2 und 100 µl Proteinase K in 25 ml Zentrifugationsröhrchen (Screw Cap Conical Tubes 25 ml, Eppendorf, Deutschland) im Freiland gesammelt und bis Ende Januar 2022 bei Raumtemperatur gelagert. Nach 10-minütiger Inkubation bei 56°C in einem Überkopfmischer (Envirogenie, Scientific Industries, Inc., USA) wurde die DNA (1800µl Lysat-Input; 120 µl Eluat in zwei Schritten je 60 µl) jeder Probe mittels des Fast Stool Mini Kits (Qiagen, Deutschland) gewonnen. Anschließend wurde zur Überprüfung der Artzugehörigkeit und des Haplotyps ein Fragment der mitochondrialen Kontrollregion mittels der Primerkombination L15995 (CTCCACTATCAGCACCCAAAG) und H16498 (CCTGAAGTAAGAACCAGATG) (Pun et al., 2009) amplifiziert und mittels Sangersequezierung analysiert. Die artspezifische STR-Analyse wurde mittels des in Cocchiararo et al. (2021) beschriebenen Marker-Sets durchgeführt. Alle 12 Proben wurden hierfür nach guter wissenschaftlicher Praxis für die Analyse von nichtinvasiv gesammelten Probenmaterials drei- bis zwölfmal repliziert (Navidi et al., 1992).

Drei der 12 Fischotter-Kotproben, die nach DNA-Extraktion Ende Januar 2022 gute Ergebnisse bei der artspezifischen STR-Analyse geliefert hatten, wurden nach zwischenzeitlich fast siebenmonatiger Lagerung (Anfang Juli 2022) in der *Collect & Extract-*Lösung bei Raumtemperatur auf Kompatibilität mit der Magnetic-Beads-Technologie mittels des EZ2 Connect-Extraktionsroboters und des EZ1&2 Virus Mini Kit v2.0 (beide Qiagen, Deutschland) getestet. Hierfür wurden gemäß programmierten Standardprotokoll je Probe 400 µl des *Collect & Extract-*Lysats als Input eingesetzt. Ab Mitte Juli 2022 wurden drei weitere Fischotter-Kotproben, die nach DNA-Extraktion Ende Januar 2022 ebenfalls gute Ergebnisse bei der artspezifischen STR-Analyse geliefert hatten, für einen beschleunigten Haltbarkeitstest sowie für die Testung auf Kompatibilität mit anderen Säulen-basierten DNA-Exraktions-Kits herangezogen und für insgesamt sieben Wochen bei 52°C gelagert. Die DNA der drei Proben wurde - entsprechend des weiter oben aufgeführten Extraktionsprotokolls für die Säulen-basierte DNA-Extraktion aus Kotproben - nach zwei, vier und sieben Wochen isoliert und mittels artspezifischer STR-Analyse (je Probe drei Replikate) auf Stabilität der Fischotter-DNA untersucht. Nach zwei Wochen Lagerung bei 52°C wurden parallel zum standardmäßig genutzten Fast Stool Mini Kit (Qiagen, Deutschland) auch DNA-Extrakte mittels der DNA-Extraktions-Kits PureLink DNA-Minikit (Thermo Fisher Scientific, Waltham, MA, USA) und NucleoSpin DNA Stool Mini kit (Macherey-Nagel, Düren, Deutschland) unter Verwendung des gleichen Protokolls (jeweils 1800 µl *Collect & Extract-*Lysat-Input pro Probe; 120 µl Eluat in zwei Schritten je 60 µl) durchgeführt. Die nach DNA-Bindung folgenden Waschschritte und die finale Elution der DNA wurde mit den Kit-spezifischen Puffern durchgeführt.

**Urin:** Acht Urinproben (bis zu 15 ml Urin-Schnee-Gemisch in 33 ml 96%igem Ethanol je Probe) von Wölfen (*Canis lupus*)*,* die im Rahmen des genetischen Wolfsmonitorings in Deutschland gesammelt wurden, wurden nach Hausknecht et al., 2007 pelletiert und in 9 ml der *Collect & Extract*-Lösung der Version 1 und 100 µl Proteinase K für eine Woche vor der DNA-Extraktion (1800µl Lysat-Input; 120 µl Eluat in zwei Schritten je 60 µl) mittels Fast Stool Mini Kit (Qiagen, Deutschland) bei Raumtemperatur gelagert und anschließend einer artspezifischen STR-Analyse (nach Jarausch et al., 2021) für nichtinvasiv gesammelte Proben unterzogen. Hierbei wurde jede Probe vierfach für drei Multiplex-Mixe ä fünf Loci repliziert.

**Wasserproben:** Im April 2021 wurden 4 Proben von jeweils einem Liter Flusswasser der Kinzig genommen und über Glasfaserfilter der Porenstärke von 2,0 µM und einem Durchmesser von 47 mm (Artikelnummer AP2504700, Merck Millipore, Deutschland) filtriert. Die vier Filter wurden unmittelbar in jeweils 5 ml der *Collect & Extract*-Lösung der Version 2 und 100 µl Proteinase K für vier Tage bei Raumtemperatur gelagert. Nach 10-minütiger Inkubation bei 56°C in einem Überkopfmischer (Envirogenie, Scientific Industries, Inc., USA) wurde die DNA (1800µl Lysat-Input; 60 µl Eluat in zwei Schritten je 30 µl) mittels QIAamp DNA Investigator Kit (Qiagen, Deutschland) extrahiert.

Jede Wasserprobe wurde mittels PCR für drei unterschiedliche Organismengruppen (Pilze, Pflanzen und Insekten; siehe Tabelle 1) amplifiziert. Eine Reaktion bestand aus 5µl SensiFAST SYBR No-ROX Kit (Biocat GmbH, Deutschland), 1,2 µl DNA-freiem Wasser, 0,4µl von den jeweiligen Vorwärts- und Rückwärtsprimern sowie 3µl DNA-Extrakt. Folgendes Themocycler-Programm wurde verwendet: 3 Minuten bei 95°C (Initiale Polymerasen-Aktivierung), 40 Zyklen von 95 °C für 5 Sekunden und 60 °C für 30 Sekunden (Denaturierung der DNA-Doppelstränge und Primerbindung bzw. gleichzeitige DNA-Kettenverlängerung) und ein letzter Schritt bei 60°C für 10 Minuten (finale Verlängerung). Nach der PCR wurden 3 µl der PCR-Produkte zur Auswertung auf ein Agarosegel unter Verwendung einer 100 bp DNA-Leiter, äquimolar (Carl Roth, Deutschland) aufgetragen.

**Tab. 1 Verwendete genetische Marker zur Untersuchung der Wasserproben**

| Taxon | Locus | Forward Primer | Reverse Primer | Referenz |
|---|---|---|---|---|
| Pflanzen | Chloroplast trnL | trnl-g GGGCAATCCTGAGCCAA | trnl-h CCATTGAGTCTCTGCACCTATC | Taberlet et al., 2007 |
| Macroinvertebraten | Cytochrom Oxidase I | fwhF2 GGDACWGGWTGAACWGTWTAYCCHCC | fwhR2n GTRATWGCHCCDGCTARWACWGG | Vamos et al., 2017 |
| Pilze | ITS | ITS3-KYO2 GATGAAGAACGYAGYRAA | ITS4 TCCTCCGCTTATTGATATGC | Brown et al. 2016 |

### Beispiel 2: Ergebnisse

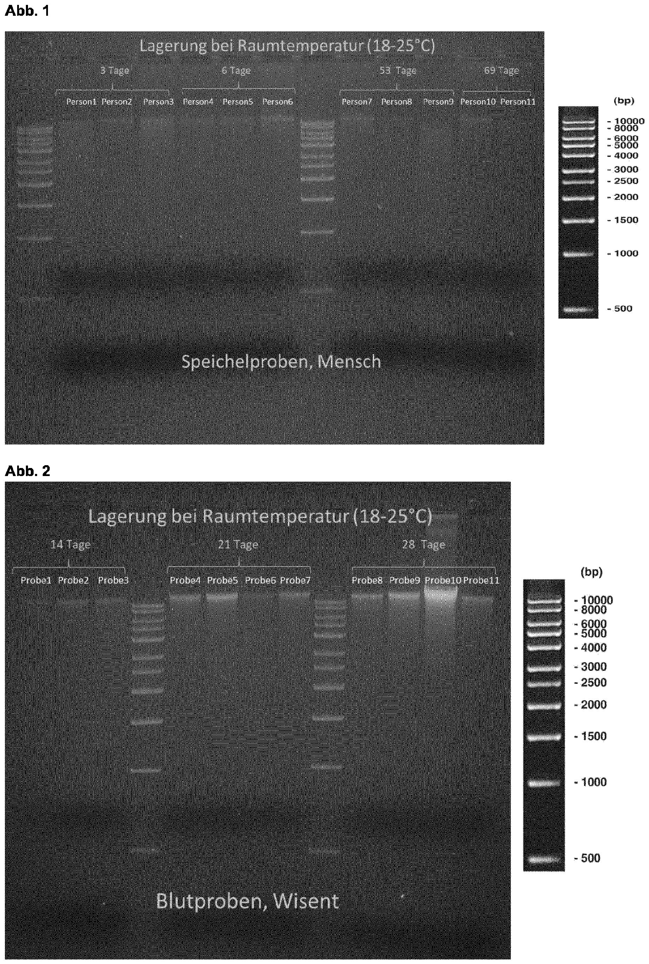

**Speichel (Mundschleimhautabstrich):** Die DNA von zwei bis drei Speichelproben der insgesamt 11 Probanden wurden nach verschiedenen Zeitabständen (nach drei, sechs, 53 und 69 Tagen) und Lagerung bei Raumtemperatur in der *Collect & Extract*-Lösung (Version 1) isoliert und deren DNA-Integrität mittels Agarosegel visualisiert (siehe Abb. 1). Die genomische DNA befindet sich zu jeder Zeit, auch nach 69 Tagen Lagerung bei Raumtemperatur in der *Collect & Extract*-Lösung, in einem hochmolekularen Zustand (bis auf Proband 9 alle über 10 kb). Die durch die Probanden selbst durchgeführte Beprobung erbrachte teilweise eine etwas geringe DNA-Ausbeute, was durch das Vorhandensein einiger schwacher DNA-Banden abgeleitet werden kann. Dies lässt sich auf ein ineffizientes Abreiben mancher Probanden ihres Mund- und Rachenraums zurückführen.

**Muskelgewebe und Haare:** Zur Überprüfung der Lysefähigkeit der *Collect & Extract-Lösung* bei härteren Probenmaterialien während der Lagerung auf Raumtemperatur wurden eine Gewebeprobe eines Wolf-Totfundes und eine Wildkatzenhaarprobe in der *Collect & Extract-*Lösung mit unterschiedlichem Ethanolanteil und unter Zugabe von 20 % einer 1M DTT-Lösung und 10 % von Proteinase K getestet. Die auf beide Versionen der *Collect & Extract-Lösung* mit unterschiedlichem Ethanolanteil verteilte Muskelgewebeprobe war nach 48 Tagen Lagerung vollständig aufgelöst. Die nachfolgende STR-Analyse war für alle getesteten Varianten erfolgreich (100 %ige Amplifikationsquote und alle Duplikate waren zueinander konsistent). Die Haarprobe, die für sechs Tage gelagert und in selbiger Konfiguration von *Collect & Extract*-Lösung der Version 2 wie die Gewebeprobe getestet wurde, war bis auf die *Collect & Extract-*Lösung ohne Ethanol vollständig gelöst. Die nachfolgende STR-Analyse war für alle getesteten Varianten erfolgreich (100 %ige Amplifikationsquote und alle Triplikate waren zueinander konsistent), also auch für die Probe mit unvollständiger Lyse (*Collect & Extract*-Lösung ohne Ethanolanteil).

**Blut:** Die DNA von drei bis vier Blutproben der insgesamt 11 Wisente wurden in verschiedenen Zeitabständen (14 Tage, 21 Tage und 28 Tage) nach Lagerung bei Raumtemperatur in der *Collect & Extract-*Lösung (Version 2) isoliert und deren DNA-Integrität mittels Agarosegel visualisiert (siehe Abb. 2).

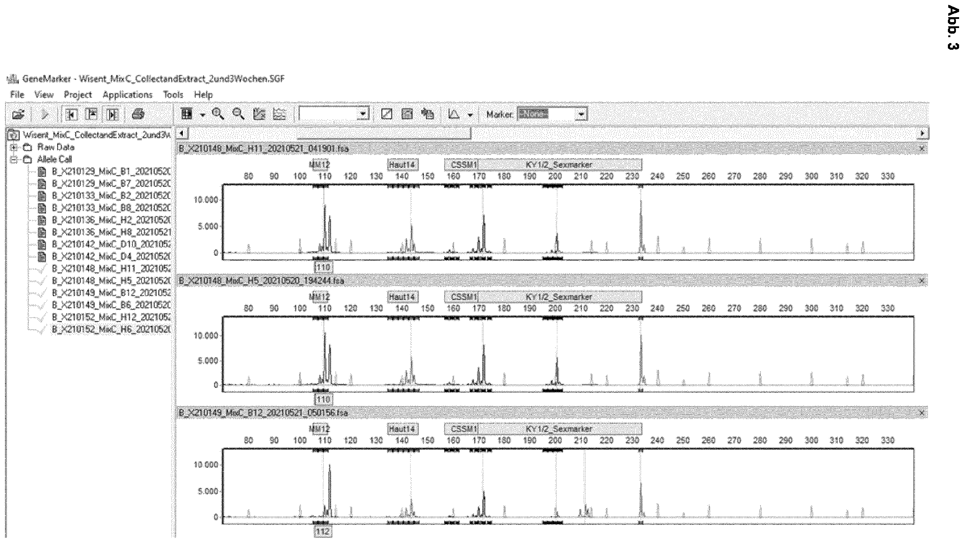

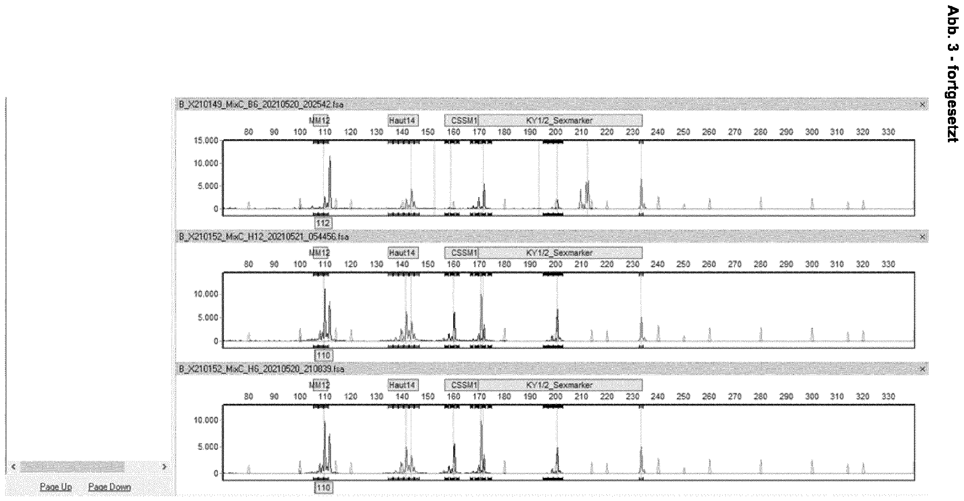

Die DNA-Integrität bleibt in der *Collect & Extract-*Lösung auch nach 28 Tagen Lagerung der Blutproben stabil. Anschließend wurden sieben Proben (14 und 21 Tage Lagerung) einer artspezifischen STR- (Abb. 3) sowie alle 11 Proben (14, 21 und 28 Tage Lagerung) einer SNP-Analyse nach Wehrenberg et al., *in prep,* unterzogen, um auf Eignung für eine weitverbreitete genetische Analysemethode, nämlich der Genotypisierung, geprüft zu werden.

Alle Proben ergaben bei beiden Genotypisierungsmethoden nahezu komplette Genotypen (Amplifikationserfolg von durchschnittlich 99%). Die genetische Geschlechtsbestimmung aller Wisente stimmte mit den bei der Blutprobenentnahme morphologisch bestimmten Geschlechtern überein.

**Kot:** Die DNA von 12 Fischotter-Kotproben, die in 15 ml der *Collect & Extract*-Lösung der Version 2 gesammelt und je nach Sammeldatum der Proben zwischen 28 bis über 40 Tage bei Raumtemperatur gelagert wurden, wurde aus 1800 µl Lsyat extrahiert. Sechs der 12 Proben (50%) ergaben bereits mit drei Replikaten für alle 21 Marker robuste, sehr gut auswertbare Daten und konnten bereits mit für Fischotterkot verhältnismäßig geringem Aufwand individualisiert werden. Alle erzeugten STR-Fragmente waren über die drei Replikate der vier Multiplex-Mixe konsistent. Eine Probe benötigte sechs Replikate und zwei weitere Proben zwölf Replikate, um die Mindestanforderung eines STR-Profils von 13 Markern für eine abgesicherte Individualisierung (Zuordnung einer Probe zu einem Fischotterindividuum) zu erreichen. Um die Allelkombination, also den Genotypen, für einen Lokus zu akzeptieren, musste ein Allel mindestens dreimal, ein mögliches zweites Allel im selben Lokus zusätzlich mindestens zweimal, innerhalb der Replikate nachgewiesen werden (nach Koelewijn et al., 2010, geringfügig modifiziert). Insgesamt wurden somit neun von 12 Fischotterkotproben erfolgreich genotypisiert. Dies entspricht einer Erfolgsquote von 75 %, die weit über der aus der Fachliteratur durchschnittlich zu erwartenden Ausbeute liegt (14% bis 73%; siehe Häjkovä et al., 2009 und darin zitierte Literatur). Die übrigen drei Proben lieferten keine ausreichenden Daten für eine Individualisierung.

Die je drei für die beschleunigte Haltbarkeits- und Kompatibilitätstestungen (Magentic-Beads- und Säulen-basierte DNA-Extraktionskits anderer Anbieter) ausgewählten Fischotter-Kotproben zeigten in allen Experimenten durchweg sehr gute Resultate. Dies spiegelt sich im folgendem Amplifikationserfolg der artspezifischen STR-Analyse wider (Durchschnitt der jeweiligen drei Proben mit jeweils drei PCR-Replikaten für 21 STR-Loci):
i) Kompatibilitätstestung mit Magnetic-Beads mittels EZ2-Connect:
   Nach nahezu sieben Monaten Lagerung bei Raumtemperatur: 93, 1 % (176 von 189).
ii) Kompatibilitätstestung mit drei Silika-Säulen-basierten DNA-Extraktionskits inkl. beschleunigtem Haltbarkeitstest bei 52°C:
   Nach über sieben Monaten Lagerung bei Raumtemperatur und anschließend zwei Wochen bei 52°C (alle drei verwendeten Kits): 99,8 % (566 von 567).
   Nach über sieben Monaten Lagerung bei Raumtemperatur und anschließend vier Wochen bei 52°C: 99, 4 % (188 von 189)
   Nach über sieben Monaten Lagerung bei Raumtemperatur und anschließend sieben Wochen bei 52°C: 100 % (189 von 189)

**Urin:** Acht Urinproben-Pellets von Wölfen wurden für eine Woche bei Raumtemperatur in der in der *Collect & Extract-*Lösung der Version 1 gelagert und nach DNA-Extraktion einer artspezifischen STR-Analyse nach Jarausch et al. (2021) unterzogen. Sechs der acht Proben ergaben für alle Loci robuste, sehr gut auswertbare Daten. Alle erzeugten STR-Fragmente waren über alle vier Replikate der drei Multiplex-Mixe konsistent. Die zwei übrigen Proben zeigten einen Amplifikationserfolg von 50 % bzw. knapp über 80 %. Sieben dieser acht erzeugten STR-Profile konnten individualisiert werden (d.h. im Rahmen des Wolfmonitoring bereits genetisch nachgewiesenen Individuen zugeordnet werden). Das entspricht einem Individualisierungserfolg von 87,5 % und liegt deutlich über der durchschnittlichen Erfolgsquote von 50-71 % (nicht veröffentlichte interne Daten von Analysen zwischen 2015 und 2021; n=573) für die Individualisierung nichtinvasiv gesammelter Urinproben im Rahmen des genetischen Wolfmonitorings in Deutschland.

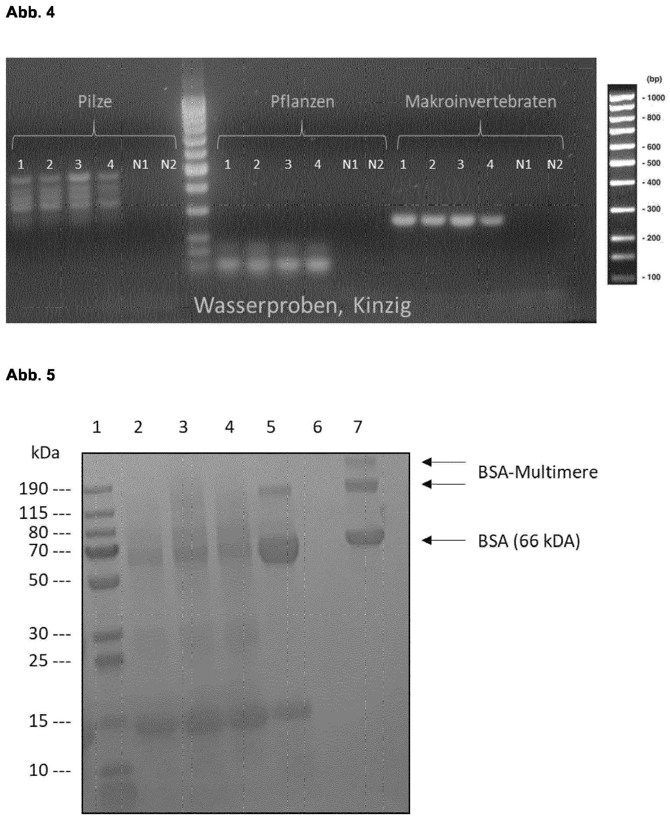

**Wasserproben:** Die DNA von vier Wasserproben wurde nach vier Tagen Lagerung in der *Collect & Extract-*Lösung (Version 2) bei Raumtemperatur extrahiert und mittels PCR für den Nachweis von drei Organismengruppen (Pilze, Pflanzen und Makroinvertebraten) amplifiziert. Zur Überprüfung der PCR wurden 3µl der PCR-Produkte auf ein Agarosegel aufgetragen (Abb. 4).

Bei allen vier Proben wurden die erwarteten Fragmentlängen (Pilze, >200 bp - ca. 600 bp; Pflanzen, 10 - 143 bp; Makroinvertebraten, 254 bp) erfolgreich amplifiziert.

### Extraktion von Proteinen:

Die Extraktion von Proteinen aus vier Wisent-Blutproben wurde nach 15 Tagen Lagerung in der *Collect & Extract*-Lösung (Version 1) durchgeführt. Sowohl die drei Proben, die bei 52°C gelagert wurden, als auch die vierte Probe sowie die Positivkontrolle (kommerziell erhältliches BSA) mit Lagerung bei Raumtemperatur zeigten nach Auftrennung mittels SDS-PAGE mehrere intakte Proteinbanden. Das vorherrschende Protein in Blutserum ist das Albumin, welches in Rinderartigen und auch in entfernt verwandten Arten zumeist eine Größe zwischen 66 und 70 kDa aufweist (Rizky et al, 2016) und in der Positivkontrolle sowie in allen vier Blutproben vorzufinden ist. Dies belegt, dass die Lagerung in *Collect & Extract-Lösung* Proteine über mindestens 15 Tage bei Raumtemperatur und bis 52°C konserviert. Bei den übrigen Banden könnte es sich hauptsächlich um Proteine der Globulin-Familie bzw. deren Untereinheiten handeln, was jedoch nicht mittels des experimentellen Setups verifiziert werden kann.

### Beispiel 3: Diskussion

Die Konservierung von biologischem Probenmaterial ist ein entscheidender Schritt für die Analyse von Biomolekülen, insbesondere bei Proben mit geringem und degradiertem Biomolekülgehalt. Auf Grundlage der hier durchgeführten Experimente wurde gezeigt, dass die Verwendung von *Collect & Extract* zur Lagerung von biologischem Probenmaterial bei Raumtemperatur auch über lange Zeiträume (mindestens für sieben Monate), aber auch bei hohen Temperaturen (nach sieben Monaten bei Raumtemperatur und anschließend 49 Tagen bei 52°C; dies entspricht nach ASTM F 1980-Norm weiteren -450 Tagen Lagerung bei Raumtemperatur) zu erfolgreichen molekularen Analysen führt. Vor allem am Beispiel der sensitiven Umweltproben mit geringen und degradierten DNA-Gehalt (Losungsproben von Fischottern und Urinproben von Wölfen) lässt sich das Potenzial der *Collect & Extract-*Lösung aufzeigen, deren Verwendung sogar überdurchschnittliche Erfolgsquoten ermöglicht hat. *Collect & Extract* ist eine kostenarm zu produzierende Lösung, die flexibel für verschiedenste Formen und Beschaffenheiten von Proben angepasst werden kann. Durch die optionale Zugabe von Chemikalien oder Reagenzien, wie 1M DTT, Proteinase K, DNase, RNAse etc., kann sie bereits vor dem Sammeln von Proben -der Zielanalyse entsprechend- modifiziert hergestellt werden. Diese Anpassungsfähigkeit kam gerade der Analyse der Fischotterkotproben zugute, die bekanntermaßen einen sehr geringen Wirts-DNA-Gehalt besitzen. Durch das Sammeln der kompletten Kotproben in je 15 ml *Collect & Extract* wurde die gesamte verfügbare DNA jeder Probe in Lösung gebracht und somit verfügbar gemacht, wodurch der Analyseerfolg im Vergleich zu publizierten Erfolgsquoten (14% bis 73%; siehe Häjkovä et al., 2009 und darin zitierte Literatur) gesteigert werden konnte. Durch die Möglichkeit Umweltproben in *Collect & Extract* zu sammeln und über lange Zeiträume unabhängig von der Umgebungstemperatur lagern zu können, werden Sammlungen für genetische Monitoringprojekte im Freiland weniger aufwendig und somit kostengünstiger und liefern zugleich durch die höheren Erfolgsquoten eine bessere Datengrundlage für die Beantwortung der wissenschaftlichen Fragestellungen, wie beispielsweise die Bestimmung der Populationsgröße von Wildtierbeständen, die Ermittlung der genetischen Populationsstruktur und Diversität bzw. des Inzuchtgrads von Populationen, für Verwandtschaftsanalysen uvm.

Die für eine Extraktion eingesetzte Menge des Überstands kann je nach durchschnittlichen Biomolekül-Gehalt variiert werden, so dass im Gegensatz zu den am Beispiel der Fischotterlosung eingesetzten 1800 µl, bei Proben mit höherem Biomolekül-Gehalt auch weniger Lysat verarbeitet oder bei Proben mit weniger Gehalt eben mehr eingesetzt werden kann. Die mittels der hier gezeigten Experimente universelle Kompatibilität der *Collect & Extract-*Lösung mit verschiedenen Extraktionskits, -Methoden und -Geräten erlaubt allen Nutzern die Weiterführung der laborspezifischen Extraktionsprotokolle, sogar mit einer Einsparung von Arbeitszeit des Laborpersonals in Form von geringerem Reagenzienmanagement und stark verkürzter Vorbehandlungen von Proben (Präparation der Ausgangsproben, Ansatz der Zelllyse und Inkubationsdauer), der Verringerung von Labormaterialienverbrauch (u.a. Reaktionsgefäßen, Pipettenspitzen und ggf. Enzyme für die Lyse von Probenmaterial), und von Geräte- (somit auch Energiekosten) und Benchkapazitäten.

Es bleibt unumstritten, dass das Kühlen bzw. Gefrieren eine sehr gut bewährte Konservierungsmethode für biologisches Material ist. Dennoch sind für diese Konservierungsmethode zumeist hohe logistische Aufwendungen zu betreiben, beispielsweise bei großangelegten Sammlungen im Freiland, um genügend Trockeneis oder Kühlgeräte über längere Zeiträume bereitzustellen. Für Laboratorien bedingt die Lagerung von Proben in Kühlgeräten oder -Räumen hohe Anschaffungs- und laufende Betriebskosten (Reparatur/Wartung/Energie/Personal), sowie die Beanspruchung von kostbarer Raum- und Laborkapazität, weshalb eine Verlagerung der Konservierung von Proben in *Collect & Extract* einen signifikanten Mehrwert bieten würde. Die Eignung der *Collect & Extract-*Lösung im medizinisch/diagnostischen oder kommerziellen Bereich, in denen die molekularen Analysen in der Regel anhand von frisch genommenen Patientenproben durchgeführt werden können, wurde durch die hier gezeigten DNA-Analysen von Speichel-, Blut-, Haar und Muskelgewebeproben sowie einer Proteinanalyse anhand von Blutproben zusätzlich bestätigt. Für das wachsende eDNA-Forschungsfeld, welches u.a. auf molekulare Analyse von andersartigen Probentypen mit zum Teil speziellen Eigenschaften, wie Gewässerfiltrate, Sedimente, Luft- und Bodenproben, angewiesen ist, ist aufgrund der flexiblen Anpassungsformel die Verwendung von *Collect & Extract* für das Sammeln von Proben sehr gut geeignet. Hier wurde gezeigt, dass *Collect & Extract* auch für die Sammlung bzw. Lagerung von Flusswasserfiltraten und die Analyse anderer Organismengruppen (Pflanzen, Pilze und Makroinvertebraten) - über Proben von Säugetieren hinaus - erfolgreich verwendet werden kann.

### Referenzen

Brown SP, Ferrer A, Dalling JW, Heath KD (2016) Don't put all your eggs in one basket: a costeffective and powerful method to optimize primer choice for rRNA environmental community analyses using the Fluidigm Access Array. Molecular Ecology Resources (2016) 16, 946-956 Camacho-Sanchez M, Burraco P, Gomez-Mestre I, Leonard JA (2013) Preservation of RNA and DNA from mammal samples under field conditions. Molecular Ecology Resources, 13(4), 663-673.
Cocchiararo B, Polednik L, Kuenzelmann B, Beran V, Nowak C (2021) Genetic assessment of the Eurasian otter population in the Ore Mountain Range. Bulletin Vydra 19: 26-35 (2021). de Groot NF, van Beers BC., Meynen G (2021) Commercial DNA tests and police investigations: a broad bioethical perspective. Journal of Medical Ethics, 47(12), 788-795. Diamandis M, White, NM, Yousef GM (2010) Personalized medicine: marking a new epoch in cancer patient management. Molecular Cancer Research, 8(9), 1175-1187.
Häjkovä P, Zemanovä B, Roche K, Hájek B (2009) An evaluation of field and noninvasive genetic methods for estimating Eurasian otter population size. Conservation Genetics 10, 1667-1681.
Hausknecht R, Gula R, Pirga B, Kuehn R (2007) Urine - a source for noninvasive genetic monitoring in wildlife. Mol Ecol Notes 7:208-212
Hupe K, Simon O (2007) Die Lockstockmethode-eine nicht invasive Methode zum Nachweis der Europäischen Wildkatze (Felis s.silvestris). Informationsdienst Naturschutz Niedersachsen 27:66-69.
Jarausch A, Harms V, Kluth G, Reinhardt I, Nowak C (2021) How the west was won: Genetic reconstruction of rapid wolf recolonization into Germany's anthropogenic landscapes. Heredity, 127(1), 92-106.
Koelewijn HP, Pérez-Haro M., Jansman H.A.H. et al. (2010) The reintroduction of the Eurasian otter (Lutra lutra) into the Netherlands: hidden life revealed by noninvasive genetic monitoring. Conservation Genetics 11, 601-614 (2010).
Navidi W, Arnheim N, Waterman MS (1992) A multiple-tubes approach for accurate genotyping of very small DNA samples by using PCR: statistical considerations. American Journal of Human Genetics 50, 347-359.
Pun KM, Albrecht C, Castella V, Fumagalli L (2009) Species identification in mammals from mixed biological samples based on mitochondrial DNA control region length polymorphism. Electrophoresis. 2009 Mar;30(6):1008-14.
Rizky N, Muhaimin R, Widodo P (2016) A comparative analysis of serum albumin from different species to determine a natural source of albumin that might be useful for human therapy. Journal of Taibah University Medical Sciences, Volume 11, Issue 3, 2016, Pages 243-249 Steyer K, Simon O, Kraus RH, Haase P, Nowak C (2013) Hair trapping with valerian-treated lure sticks as a tool for genetic wildcat monitoring in low-density habitats. European Journal of Wildlife Research, 59(1), 39-46.
Wehrenberg G, Tokarska M, Cocchiararo B, Nowak C (2021) A reduced SNP panel for noninvasive genetic assessment of a genetically impoverished species, the European bison (Bos bonasus), in preparation
Thomsen PF & Willerslev E (2015) Environmental DNA-An emerging tool in conservation for monitoring past and present biodiversity. Biological conservation, 183, 4-18.
Taberlet P, Waits LP, Luikart G (1999) Noninvasive genetic sampling: look before you leap. Trends in ecology & evolution, 14(8), 323-327.
Taberlet P, Bonin A, Zinger L, Coissac E (2018) Environmental DNA: For Biodiversity Research and Monitoring. Oxford: Oxford University Press.
Vamos E, Elbrecht V, Leese, F (2017) Short COI markers for freshwater macroinvertebrate metabarcoding. Metabarcoding and Metagenomics, 1, e14625.

## Patentansprüche

1. Lösung zur Stabilisierung von Biomolekülen, die
ein Guanidinsalz, bevorzugt 100 mM und 800 mM Guanidinsalz, am meisten bevorzugt 250 mM bis 400 mM;
Natriumlaurylsulfat oder N-Laurylsarcosin, bevorzugt 0,1 bis 2,5 Gew.-% Natriumlaurylsulfat oder N-Laurylsarcosin, am meisten bevorzugt 0,8 bis 1,2 Gew.-% Natriumlaurylsulfat oder N-Laurylsarcosin; und
gegebenenfalls Ethanol, bevorzugt 3 bis 50 Vol.%, am meisten bevorzugt 4,5 bis 35,0 Vol.%
umfasst.

2. Lösung zur Stabilisierung von Biomolekülen nach Anspruch 1, wobei die Lösung eine wässrige Lösung ist,

3. Lösung zur Stabilisierung von Biomolekülen nach Anspruch 1 oder 2, wobei das Guanidinsalz Guanidinhydrochlorid oder Guanidinthiocyanat ist.

4. Lösung zur Stabilisierung von Biomolekülen nach einem der Ansprüche 1 bis 3, wobei die Lösung weiterhin einen Puffer, bevorzugt Tris-(hydroxymethyl)-aminomethanhydrochlorid (TRIS HCl) umfasst.

5. Lösung zur Stabilisierung von Biomolekülen nach einem der Ansprüche 1 bis 4, wobei die Lösung weiterhin einen Chelator, bevorzugt Ethylendinitrilotetraessigsäure (EDTA) umfasst.

6. Lösung zur Stabilisierung von Biomolekülen nach einem der Ansprüche 1 bis 5, wobei die Lösung weiterhin ein oder mehrere Detergenzien zur Zelllyse umfasst, bevorzugt Tween 20 und/oder Triton X-100.

7. Lösung zur Stabilisierung von Biomolekülen nach einem der Ansprüche 1 bis 6, die weiterhin eine Proteinase, bevorzugt Proteinase K umfasst.

8. Lösung zur Stabilisierung von Biomolekülen nach Anspruch 7, wobei die Lösung Proteinase K in einer Menge von 2 bis 100 mAU/ml Lösung, bevorzugt 4 bis 80 mAU/ml Lösung und am meisten bevorzugt etwa 60 mAU/ml Lösung enthält.

9. Lösung zur Stabilisierung von Biomolekülen nach einem der Ansprüche 1 bis 8, die weiterhin ein Reagenz zur Reduzierung der Disulfide umfasst, bevorzugt, Dithiothreitol (DTT) oder Mercaptoethanol.

10. Lösung zur Stabilisierung von Biomolekülen nach Anspruch 9, wobei die Lösung 1M DTT oder 14,3 M Mercaptoethanol in einer Menge von bis zu 20 Vol.% umfasst.

11. Verfahren zur Stabilisierung von Biomolekülen, umfassend
(a) die Zugabe der Biomoleküle in eine Lösung nach einem der Ansprüche 1 bis 10, und
(b) gegebenenfalls die Lagerung der Lösung mit den Biomolekülen bei -273°C bis 70°C, bevorzugt bei -20°C bis 30°C, bevorzugt für mindestens 14 Tage, mehr bevorzugt mindestens 1 Monat und am meisten bevorzugt für mindestens 6 Monate.

12. Verfahren nach Anspruch 11, wobei im Schritt (a) der Lösung Zellen zugegeben werden, die die Biomoleküle umfassen.

13. Verfahren nach Anspruch 13, wobei die Zellen Teil einer biologischen Probe sind und die biologische Probe bevorzugt Blut, Plasma, Serum, Urin, Speichel, Lymphe, Liquor, Sperma, Haut (-schuppen), Stuhl, Haare, Gewebe, Wasser, Pflanzenmaterial oder Bodenmaterial ist.

14. Lösung nach einem der Ansprüche 1 bis 10 oder Verfahren nach einem der Ansprüche 11 bis 13, wobei die Biomoleküle DNA, RNA und Proteine umfassen.

15. Kit, umfassend
die Lösung nach einem der Ansprüche 1 bis 10,
ein oder mehrere Gefäße, bevorzugt ein oder mehrere Röhrchen, die fest verschlossen werden können, und
gegebenenfalls Instruktionen zur Verwendung der Kits.
